(19) Europäisches Patentamt
European Patent Office
Office européen des brevets

(11) Numéro de publication: **0 319 983 B1**

(12) # FASCICULE DE BREVET EUROPEEN

(45) Date de publication de fascicule du brevet: **03.06.92**

(51) Int. Cl.$^{5}$: **A61K 7/06**

(21) Numéro de dépôt: **88120553.8**

(22) Date de dépôt: **08.12.88**

Le dossier contient des informations techniques présentées postérieurement au dépôt de la demande et ne figurant pas dans le présent fascicule.

(54) **Association à base de dérivés de pyrimidine et d'antagonistes de calcium en vue d'induire et de stimuler la croissance des cheveux et diminuer leur chute.**

(30) Priorité: **09.12.87 LU 87068**

(43) Date de publication de la demande:
**14.06.89 Bulletin 89/24**

(45) Mention de la délivrance du brevet:
**03.06.92 Bulletin 92/23**

(84) Etats contractants désignés:
**AT BE CH DE FR GB IT LI NL SE**

(56) Documents cités:
**BE-A- 905 375**
**GB-A- 2 177 918**
**US-A- 4 139 619**

(73) Titulaire: **L'OREAL**
**14, Rue Royale**
**F-75008 Paris(FR)**

(72) Inventeur: **Grollier, Jean Francois**
**16 bis boulevard Morland**
**F-75004 Paris(FR)**
Inventeur: **Rosenbaum, Georges**
**2 Rue J.H. Mansart**
**F-92600 Asnières(FR)**

(74) Mandataire: **Casalonga, Axel et al**
**BUREAU D.A. CASALONGA - JOSSE Morassistrasse 8**
**W-8000 München 5(DE)**

EP 0 319 983 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet une nouvelle association d'agents antagonistes de calcium et de dérivés de pyrimidine en vue d'induire et de stimuler la croissance des cheveux et diminuer leur chute ainsi qu'un procédé de traitement mettant en oeuvre une telle association.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle dit "cycle pilaire" au cours duquel le cheveu se forme, croît et tombe avant d'être remplacé par un nouveau cheveu qui apparaît dans le même follicule.

On observe, au cours d'un cycle pilaire, successivement trois phases, à savoir : la phase anagène, la phase catagène et la phase télogène.

Au cours de la première phase, dite anagène, le cheveu passe par une période de croissance active associée à une intense activité métabolique au niveau du bulbe.

La deuxième phase, dite catagène, est transitoire et elle est marquée par un ralentissement des activités mitotiques. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber, poussé par un cheveu anagène naissant.

Ce processus de renouvellement physique permanent subit une évolution naturelle au cours du vieillissement, les cheveux deviennent plus fins et leurs cycles plus courts.

L'alopécie survient lorsque ce processus de renouvellement physique est accéléré ou perturbé, c'est-à-dire lorsque les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté qui peut conduire à la calvitie.

On a déjà préconisé pour le traitement de la chute des cheveux l'amino-6 dihydro-1,2 hydroxy-1 imino-2 pipéridino-4 pyrimidine ou "Minoxidil" dans des compositions topiques permettant de réduire ou de supprimer l'effet de l'alopécie et d'induire et de stimuler la croissance des cheveux et diminuer leur chute.

On connaît, par ailleurs, en thérapeutique, par voie orale ou parentérale, des antagonistes de calcium, en particulier dans le traitement des maladies cardiovasculaires.

On sait également que la nifédipine, qui est un antagoniste de calcium, est utilisée comme l'alternative du minoxidil pour la repousse des cheveux et pour le traitement topique de l'alopécie, comme l'enseigne la demande de brevet BE-A 905 375.

La demanderesse a découvert, de façon surprenante, qu'en associant des antagonistes de calcium qui en eux-mêmes n'ont pas de propriété au niveau de l'induction de la stimulation des cheveux ainsi qu'au niveau du freinage de leur chute, avec certains dérivés de pyrimidine, on constate de façon surprenante une induction et une stimulation améliorées de la croissance des cheveux et une action plus forte sur le freinage de la chute.

On a constaté, en particulier, une mise en action plus rapide de l'association par rapport aux composés isolés. Grâce à l'association, on peut également utiliser le dérivé de pyrimidine à une concentration plus faible.

L'efficacité ou la rapidité d'action d'une composition de traitement de l'alopécie se détermine plus particulièrement grâce au trichogramme et en particulier au photo-trichogramme permettant de déterminer, entre autre, le pourcentage de cheveux en phase anagène par rapport aux cheveux en phase télogène.

L'association conforme à l'invention permet en particulier d'améliorer ce pourcentage par rapport aux composés utilisés séparément.

Un objet de l'invention est donc constitué par l'association d'antagonistes de calcium avec des dérivés de pyrimidine en vue d'induire ou de stimuler la croisance des cheveux et diminuer leur chute.

Un autre objet de l'invention est constitué par des compositions cosmétiques et/ou pharmaceutiques contenant ces composés.

L'invention a également pour objet des dispositifs à plusieurs compartiments contenant l'association citée ci-dessus.

D'autres objets de l'invention apparaîtront à la lecture de la description et des exemples qui suivent.

L'association conforme à l'invention est essentiellement caractérisée par le fait qu'elle comprend :

a/ un composant (A) comprenant, dans un milieu physiologiquement acceptable, au moins un antagoniste de calcium, et

b/ un composant (B) contenant dans un milieu physiologiquement acceptable, au moins un dérivé de pyrimidine répondant à la formule :

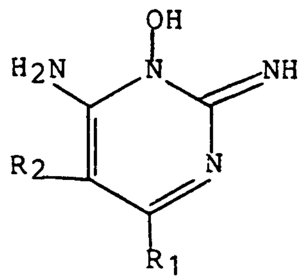

(I)

dans laquelle $R_1$ désigne un groupement

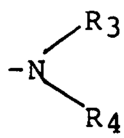

,

$R_3$ et $R_4$, indépendamment l'un de l'autre, désignant hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, en vue d'induire et stimuler la croissance des cheveux et diminuer leur chute.

Les agents antagonistes de calcium sont plus particulièrement choisis parmi les dérivés de la papavérine, les dérivés de la 1,4-dihydropyridine, les dérivés de la benzothiazépine, les dérivés des cinnamylpipérazines, ainsi que la nicergoline et le bépridil.

Les dérivés de papavérine sont en particulier choisis parmi le vérapamil ou bis(diméthoxy-3,4 phényl)-1,7 méthyl-5 isopropyl-1 cyano-1 aza-5 heptane et son chlorhydrate et le gallopamil ou [[(diaméthoxy-3,5 phényl)-2 éthyl] méthylamino]-5 isopropyl-2 (triméthoxy-3,4,5 phényl)-2 valéronitrile.

Les dérivés de 1,4-dihydropyridine sont plus particulièrement choisis parmi la nifédipine ou 4-(2′nitrophényl)-2,6-diméthyl-3,5-dicarbométhoxy 1,4-dihydropyridine; la nicardipine ou 4-(3′-nitrophényl)-2,6-diméthyl-3-carbométhoxy-5-(méthylbenzylamino) carboxyéthoxy 1,4-dihydropyridine et son chlorhydrate; la nimodipine ou 4-(3′nitrophényl-2,6-diméthyl-3-carbo (2-méthoxyéthoxy)-5-carboisopropoxy 1,4-dihydropyridine; la niludipine ou 4-(3′nitrophényl)-2,6-diméthyl 3,5-dicarbo 2-propoxyéthoxy 1,4-dihydropyridine; la félodipine ou 4-(2′,3′-dichlorophényl)-2,6-diméthyl-3-carboéthoxy 5-carbométhoxy 1,4-dihydropyridine; la nitrendipine ou 4-(3′-nitrophényl)-2,6-diméthyl 3-carboéthoxy 5-carbométhoxy 1,4-dihydropyridine; la nilvadipine ou 4-(3′-nitrophényl) 2-cyano 6-méthyl 3-carbométhoxy 5-carbo-(1-méthyléthoxy) 1,4-dihydropyridine; 1'isradipine ou 4-(4′-benzofurazanyl, 2,6-diméthyl 3-carbométhoxy 5-carbo-(1-méthyléthoxy) 1,4-dihydropyridine; la nisoldipine ou 4-(2′-nitrophényl) 2,6-diméthyl 3-carboisobutoxy 5-carbométhoxy 1,4-dihydropyridine.

Les dérivés de benzothiazépine sont constitués plus particulièrement par le Diltiazem ou 3-(acétyloxy) 5- [2-(diméthylamino)-éthyl] 2,3-dihydro-2-(4-méthoxyphényl) 1,5-benzothiazépine-4 (5H) one et son chlorhydrate.

Les cinnamylpipérazines sont plus particulièrement choisies parmi la Cinnarizine ou 1-cinnamyl-4-diphénylméthylpipérazine, ses chlorhydrate et clofibrate.

La Flunarizine ou 1-cinnamyl 4- di [(p.-fluorophényl) méthyl] pipérazine et son dichlorhydrate.

La Nicergoline est plus particulièrement constituée par le bromo-5 nicotinate de (10-méthoxy 1,6-diméthylergoline)8-méthanol.

Le Bepridil est plus particulièrement constitué par le 1-isobutoxy 2-pyrrolidino-3-N-benzyl anilinopropane.

Les agents antagonistes de calcium plus particulièrement préférés sont le Vérapamil, la Nifédipine, la Nicardipine, le Diltiazem, la Cinnarizine et la Flunarizine.

Dans l'association conforme à l'invention et pour les composés de formule (I), le groupement alkyle ou alcoxy désigne de préférence un groupement ayant 1 à 4 atomes de carbone; le groupement alcényle désigne de préférence un groupement ayant 2 à 5 atomes de carbone; le groupement aryle désigne de préférence un groupement phényle et le groupement cycloalkyle désigne de préférence un groupement ayant 4 à 6 atomes de carbone.

Les composés particulièrement préférés de formule (I) sont choisis parmi les composés dans lesquels R2 désigne hydrogène et $R_1$ désigne un groupement :

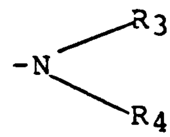

dans lequel $R_3$ et $R_4$ forment un cycle pipéridinyle, ainsi que leurs sels tels que, par exemple, le sulfate.

Parmi ces composés, le composé particulièrement préféré est constitué par l'amino-6 dihydroxy-1,2 hydroxy-1 imino-2 pipéridino-4 pyrimidine, encore appelé Minoxidil.

L'agent antagoniste de calcium est utilisé de préférence dans le composant (A) dans des proportions comprises entre 0,01 et 10% en poids, et en particulier entre 0,01 et 5% en poids et plus particulièrement entre 0,1 et 3% en poids, alors que le dérivé de pyrimidine de formule (I) est utilisé de préférence dans le composant (B) dans des proportions comprises entre 0,05 et 10% en poids, et de préférence entre 0,05 et 5% en poids, et en particulier entre 0,5 et 4% en poids.

Lorsque les composants (A) et (B) sont utilisés dans une composition unique, la proportion en agents antagonistes de calcium est comprise de préférence entre 0,01 et 5% en poids par rapport au poids total de la composition, et de préférence entre 0,05 et 3%; le dérivé de pyrimidine de formule (I) est utilisé dans les compositions dans une proportion comprise entre 0,05 et 6% en poids par rapport au poids total de la composition, et de préférence entre 0,1 et 5%, et en particulier entre 0,5 et 2% en poids.

Le milieu physiologiquement acceptable pour les composants (A) et (B) est un milieu utilisable en pharmacie et en cosmétique, et peut être constitué normalement par de l'eau, un mélange d'eau et d'un ou plusieurs solvants organiques ou par un mélange de solvants organiques cosmétiquement ou pharmaceutiquement acceptables.

Les solvants utilisables sont choisis plus particulièrement parmi les alcools inférieurs en $C_1$-$C_4$ tels que l'alcool éthylique, l'alcool isopropylique, l'alcool tertiobutylique, les alkylèneglycols comme le propylèneglycol, les alkyléthers de mono- et de dialkylèneglycol et plus particulièrement le monoéthyléther d'éthylèneglycol, le monométhyléther de propylèneglycol, le monoéthyléther de diéthylèneglycol.

Ces solvants, lorsqu'ils sont utilisés dans un milieu aqueux, sont présents de préférence dans des proportions comprises entre 1 et 80% en poids par rapport au poids total de la composition.

Les milieux physiologiquement acceptables peuvent être épaissis ou non. On utilise dans ce but des agents épaississants ou gélifiants bien connus dans l'état de la technique, tels que plus particulièrement les hétérobiopolysaccharides comme la gomme de xanthane ou les scléroglucanes, les dérivés de cellulose, les polymères acryliques réticulés ou non.

Les épaississants sont présents de préférence dans des proportions comprises entre 0,1 et 5% et en particulier entre 0,4 et 3% en poids par rapport au poids total de chacun des composants lorsqu'ils sont utilisés de façon séparée ou par rapport au poids total de la composition contenant les composants (A) et (B).

Les compositions constituées, soit par les composants (A) et (B), soit par la composition contenant les deux composants (A) et (B), peuvent également contenir tous autres adjuvants habituellement utilisés dans les compositions destinées à une application topique à utilisation cosmétique ou pharmaceutique, et plus particulièrement des agents conservateurs, des agents complexants, des colorants, des agents alcalinisants ou acidifiants, des agents tensio-actif anioniques, cationiques, non ioniques, amphotères ou leurs mélanges, des polymères anioniques, cationiques, non ioniques ou amphotères, ainsi que leurs mélanges.

Le pH de ces compositions lorsqu'elles contiennent de l'eau, peut varier entre 4 et 9.

Ces compositions peuvent également être conditionnées sous pression dans un dispositif aérosol.

Dans le composant (B), les dérivés de pyrimidine de formule (I) peuvent être présents, soit sous forme dissoute dans le milieu physiologiquement acceptable, ou alors en totalité ou partiellement en suspension dans ce milieu, en particulier sous forme de particules ayant une granulométrie inférieure à 80 µm, de préférence inférieure à 20 µm, et en particulier inférieure à 5 µm.

Une première forme de réalisation de l'invention consiste à utiliser l'association définie ci-dessus sous

forme d'une composition unique contenant les composants (A) et (B).

Une forme particulièrement préférée de l'invention consiste à conserver dans des dispositifs séparés les composants (A) et (B) et à préparer la composition contenant l'agent antagoniste du calcium et les dérivés de pyrimidine de formule (I) de façon extemporanée tout juste avant application.

Une autre forme de réalisation consiste enfin à appliquer les composants (A) et (B) de façon séparée, soit simultanément, soit de façon successive ou décalée dans le temps.

L'association conforme à l'invention peut être conditionnée dans ce cas, en particulier, dans un dispositif à plusieurs compartiments encore appelé "kit" ou nécessaire, dont le premier compartiment contient le composant (A) renfermant l'agent antagoniste de calcium et le second compartiment contient le composant (B) à base du dérivé de pyrimidine de formule (I). Ce dispositif peut être équipé d'un moyen de mélange des compositions tout juste au moment de l'application.

Le traitement pour induire et stimuler la croissance des cheveux et diminuer leur chute consiste principalement à appliquer sur les zones alopéciques du cuir chevelu et les cheveux d'un individu, l'association telle que définie ci-dessus, soit au moyen d'une composition unique, soit par application des composants (A) et (B) ou (B) et (A), de façon successive ou décalée dans le temps.

Un mode d'application préféré consiste à appliquer 1 à 5 g de la composition ou de chacun des composants (A) et (B) sur la zone alopécique à une fréquence de une à deux applications par jour pendant 1 à 7 jours par semaine et suivant une durée de 1 à 6 mois.

Le procédé de traitement présente les caractéristiques d'un traitement thérapeutique dans la mesure où l'association conforme à l'invention a une activité thérapeutique au niveau des mécanismes biologiques du cycle pilaire et du dysfonctionnement de celui-ci.

Un objet de l'invention est donc constitué par l'utilisation de l'association telle que définie ci-dessus pour la préparation d'un médicament ayant pour effet d'induire ou de stimuler la croissance des cheveux et de freiner leur chute.

Le procédé conforme à l'invention présente par ailleurs les caractéristiques d'un procédé de traitement cosmétique dans la mesure où il permet d'embellir les cheveux ou le cuir chevelu.

Les exemples suivants sont destinés à illustrer l'invention sans pour autant présenter un caractère limitatif.

EXEMPLE 1

On prépare une lotion de composition suivante :

| | |
|---|---|
| - Vérapamil chlorhydrate | 0,60 g |
| - Minoxidil | 1,00 g |
| - Propylèneglycol | 20,00 g |
| - Alcool éthylique | 50,00 g |
| - Eau | qsp 100,00 g |

EXEMPLE 2

On prépare une lotion de composition suivante :

| | |
|---|---|
| - Diltiazem chlorhydrate | 0,90 g |
| - Minoxidil | 1,00 g |
| - Alcool éthylique | 95,00 g |
| - Propylèneglycol | qsp 100,00 g |

EXEMPLE 3

On prépare une lotion de composition suivante :

| | |
|---|---|
| - Nifédipine | 0,15 g |
| - Minoxidil | 1,00 g |
| - Propylèneglycol | 20,00 g |
| - Alcool éthylique | 50,00 g |
| - Eau | qsp 100,00 g |

EXEMPLE 4

On conditionne en kit deux compositions (A) et (B) renfermant respectivement :

| Composition (A) : | |
|---|---|
| - Flunarizine dichlorhydrate | 0,20 g |
| - Alcool éthylique | 95,00 g |
| - Propylèneglycol | qsp 100,00 g |

| Composition (B) : | |
|---|---|
| - Minoxidil | 3,00 g |
| - Propylèneglycol | 20,00 g |
| - Alcool éthylique | 50,00 g |
| - Eau | qsp 100,00 g |

On applique le mélange extemporané des compositions (A) et (B).

EXEMPLE 5

On conditionne en kit deux compositions (A) et (B) renfermant respectivement :

| Composition (A) : | |
|---|---|
| - Nicardipine chlorhydrate | 0,60 g |
| - Propylèneglycol | 20,00 g |
| - Alcool éthylique | 50,00 g |
| - Eau | qsp 100,00 g |

| Composition (B) : | |
|---|---|
| - Minoxidil micronisé, dont les particules ont un diamètre moyen inférieur à 2 µm | 2,00 g |
| - Acide polyacrylique réticulé PM = 3 millions, vendu sous la dénomination "CARBOPOL 934" par la Société GOODRICH | 1,00 g |
| - Propylèneglycol | 4,50 g |
| - Amino-2 méthyl-2 propanol-1 | qs pH 7 |
| - Conservateur | qs |
| - Eau | qsp 100,00 g |

On applique les compositions en succession décalée dans le temps, la composition (A) le matin, la composition (B) le soir.

EXEMPLE 6

On conditionne en kit deux compositions (A) et (B) renfermant respectivement :

| Composition (A) : | |
|---|---|
| - Cinnarizine | 0,50 g |
| - Alcool éthylique | 95,00 g |
| - Propylèneglycol | qsp 100,00 g |

| Composition (B) : | |
|---|---|
| - Minoxidil | 2,00 g |
| - Alcool éthylique | 95,00 g |
| - Propylèneglycol | qsp 100,00 g |

On applique les compositions en succession décalée dans le temps, en alternance journalière : 1er jour, la composition (A), 2ème jour, la composition (B).

On constate pour chacun des exemples 1 à 6 après application de la (ou des) composition(s) sur les parties alopéciques du cuir chevelu, à raison d'un traitement journalier sur 3 mois, une amélioration sensible du rapport :

$$\frac{\text{anagène}}{\text{télogène}}$$

EXEMPLE 7

On prépare une lotion de composition suivante :

| | |
|---|---|
| - Diltiazem | 3,00 g |
| - Minoxidil | 0,54 g |
| - Propylèneglycol | 20,00 g |
| - Alcool éthylique | 50,00 g |
| - Eau | qsp 100,00 g |

EXEMPLE 8

On prépare une lotion de composition suivante :

| | |
|---|---|
| - Cinnarizine | 3,00 g |
| - Minoxidil | 0,54 g |
| - Propylèneglycol | 20,00 g |
| - Alcool éthylique | 50,00 g |
| - Eau | qsp 100,00 g |

**Revendications**

**1.** Association destinée à induire et à stimuler la croissance des cheveux et diminuer leur chute, caractérisée par le fait qu'elle comprend :
a/ un composant (A) contenant dans un milieu physiologiquement acceptable, au moins un agent antagoniste de calcium; et
b/ un composant (B) contenant dans un milieu physiologiquement acceptable, au moins un dérivé de

pyrimidine, répondant à la formule :

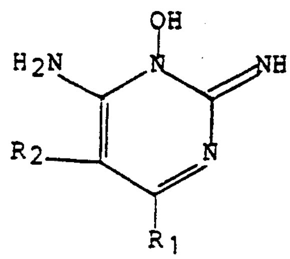

(I)

dans laquelle $R_1$ désigne un groupement

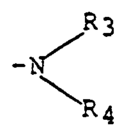

,

$R_3$ et $R_4$, indépendamment l'un de l'autre, désignant hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

2. Association selon la revendication 1, caractérisée par le fait que les antagonistes de calcium sont choisis parmi les dérivés de Papavérine, les dérivés de 1,4-dihydropyridine, les dérivés de benzothiazépine, les dérivés de cinnamylpipérazine, la Nicergoline et le Bepridil.

3. Association selon la revendication 2, caractérisée par le fait que les dérivés de Papavérine sont choisis parmi le Vérapamil, le Gallopamil; que les dérivés de 1,4-dihydropyridine sont choisis parmi la Nifédipine, la Nicardipine, la Nimodipine, la Niludipine, la Félodipine, la Nitrendipine, la Nisoldipine, la Nilvadipine, l'Isradipine et les dérivés de benzothiazépine tel que le Diltiazem; que les dérivés de cinnamylpipérazine sont choisis parmi la Cinnarizine et la Flunarizine.

4. Association selon l'une quelconque des revendications 1 à 3, caractérisée par le fait que le dérivé de pyrimidine de formule (I) est choisi parmi les composés de formule (I), dans laquelle $R_2$ désigne un hydrogène et $R_1$ représente un groupement :

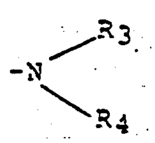

,

dans lequel $R_3$ et $R_4$ forment un cycle pipéridinyle, ainsi que leurs sels.

5. Association selon la revendication 4, caractérisée par le fait que le dérivé de pyrimidine de formule (I) est constitué par l'amino-6 dihydro-1,2 hydroxy-1 imino-2 pipéridino-4 pyrimidine ou Minoxidil.

6. Association selon l'une quelconque des revendications 1 à 5, caractérisée par le fait que l'antagoniste

de calcium est utilisé dans le composant (A) dans des proportions comprises entre 0,01 et 10% en poids, et de préférence entre 0,01 et 5% en poids, et que le dérivé de pyrimidine de formule (I) est utilisé dans le composant (B) dans des proportions comprises entre 0,05 et 10% en poids, de préférence entre 0,05 et 5% en poids, et en particulier entre 0,5 et 4% en poids.

**7.** Association selon l'une quelconque des revendications 1 à 6, caractérisée par le fait que les composants (A) et (B) sont présents dans une composition unique, dans laquelle l'agent antagoniste de calcium est présent dans des proportions comprises entre 0,01 et 5% en poids, par rapport au poids total de la composition, et de préférence entre 0,05 et 3% en poids, et que le dérivé de pyrimidine de formule (I) est présent dans des proportions comprises entre 0,05 et 6% en poids, par rapport au poids total de la composition, de préférence entre 0,1 et 5%, et en particulier entre 0,5 et 2% en poids.

**8.** Association selon l'une quelconque des revendications 1 à 7, caractérisée par le fait que le milieu physiologiquement acceptable pour les composants (A) et (B) est un milieu constitué par de l'eau ou un mélange d'eau et d'un ou plusieurs solvants organiques ou par un mélange de solvants organiques, cosmétiquement ou pharmaceutiquement acceptables.

**9.** Association selon l'une quelconque des revendications 1 à 8, caractérisée par le fait que l'un au moins des composants (A) ou (B) contient un solvant choisi parmi les alcools inférieurs en $C_1$-$C_4$, les alkylèneglycols, les alkyléthers de mono- et de dialkylèneglycol.

**10.** Association selon l'une quelconque des revendications 1 à 9, caractérisée par le fait que l'un au moins des composants (A) ou (B) est épaissi au moyen d'agents épaississants ou gélifiants.

**11.** Association selon l'une quelconque des revendications 1 à 10, caractérisée par le fait que l'un au moins des composants (A) ou (B) contient également un adjuvant cosmétiquement ou pharmaceutiquement acceptable choisi parmi des agents conservateurs, des agents complexants, des colorants, des agents alcalinisants ou acidifiants, des agents tensio-actifs, anioniques, cationiques, non ioniques ou amphotères, ainsi que leurs mélanges, des polymères anioniques, cationiques, non ioniques ou amphotères, ainsi que leurs mélanges.

**12.** Procédé de traitement cosmétique des cheveux ou du cuir chevelu, caractérisé par le fait que l'on applique les composants (A) et (B) selon une association telle que définie dans l'une quelconque des revendications 1 à 11, au moyen d'une composition unique ou de façon séparée, soit simultanément, soit de façon successive ou décalée dans le temps, en vue d'embellir les cheveux ou le cuir chevelu.

**13.** Dispositif à plusieurs compartiments ou "kit", caractérisé par le fait qu'il comprend dans un premier compartiment un composant (A) tel que défini dans l'une quelconque des revendications 1 à 10, et un second compartiment contenant le composant (B) tel que défini dans l'une quelconque des revendications 1 à 10.

**14.** Association selon l'une quelconque des revendications 1 à 10 pour son application comme médicament pour le traitement thérapeutique de la chute des cheveux.

**15.** Utilisation de l'association selon l'une quelconque des revendications 1 à 10 pour la préparation d'un médicament destiné à être utilisé dans le traitement thérapeutique de la chute des cheveux.

**Claims**

**1.** Combination intended for inducing and stimulating hair growth and reducing hair loss, characterised in that it comprises:

a/ a component (A) containing, in a physiologically acceptable medium, at least one calcium antagonist agent; and

b/ a component (B) containing, in a physiologically acceptable medium, at least one pyrimidine derivative corresponding to the formula:

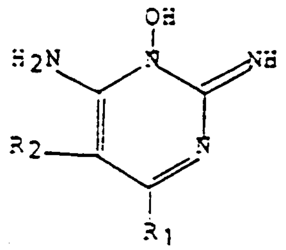

(I)

in which $R_1$ denotes a group

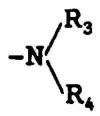

$R_3$ and $R_4$, independently of one another, denoting hydrogen or an alkyl, alkenyl, alkylaryl or cycloalkyl group, $R_3$ and $R_4$ can also form a heterocycle with the nitrogen atom to which they are linked, chosen from aziridinyl, azetidinyl, pyrrolidinyl, piperidyl, hexahydroazepinyl, heptamethylenimine, octamethylenimine, morpholine and 4-alkylpiperazinyl groups, it being possible for the heterocyclic groups to be substituted on the carbon atoms with one to three lower alkyl, hydroxyl or alkoxy groups; the group $R_2$ is chosen from a hydrogen atom and an alkyl, alkenyl, alkylalkoxy, cycloalkyl, aryl, alkylaryl, arylalkyl, alkylarylalkyl, alkoxyarylalkyl or haloarylalkyl group; as well as the addition salts with physiologically acceptable acids, the components (A) and (B) forming part of one and the same composition or being intended for use separately, either simultaneously or successively or separated by an interval of time, on the scalp or the hair.

**2.** Combination according to Claim 1, characterised in that the calcium antagonists are chosen from papaverine derivatives, 1,4-dihydropyridine derivatives, benzothiazepine derivatives, cinnamylpiperazine derivatives, nicergoline and bepridil.

**3.** Combination according to Claim 2, characterised in that the papaverine derivatives are chosen from verapamil and gallopamil; in that the 1,4-dihydropyridine derivatives are chosen from nifedipine, nicardipine, nimodipine, niludipine, felodipine, nitrendipine, nisoldipine, nilvadipine and isradipine; in that the benzothiazepine derivative consists of diltiazem; and in that the cinnamylpiperazine derivatives are chosen from cinnarizine and flunarizine.

**4.** Combination according to any one of Claims 1 to 3, characterised in that the pyrimidine derivative of formula (I) is chosen from the compounds of formula (I) in which $R_2$ denotes a hydrogen and $R_1$ represents a group:

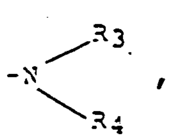

in which $R_3$ and $R_4$ form a piperidyl ring, as well as their salts.

**5.** Combination according to Claim 4, characterised in that the pyrimidine derivative of formula (I) consists of 6-amino-1,2-dihydro-1-hydroxy-2-imino-4-piperidino-pyrimidine or minoxidil.

**6.** Combination according to any one of Claims 1 to 5, characterised in that the calcium antagonist is used in the component (A) in proportions of between 0.01 and 10% by weight, and preferably between 0.01 and 5% by weight, and in that the pyrimidine derivative of formula (I) is used in the component (B) in proportions of between 0.05 and 10% by weight, preferably between 0.05 and 5% by weight, and especially between 0.5 and 4% by weight.

**7.** Combination according to any one of Claims 1 to 6, characterised in that the components (A) and (B)

are present in a single composition in which the calcium antagonist agent is present in proportions of between 0.01 and 5% by weight relative to the total weight of the composition, and preferably between 0.05 and 3% by weight, and in that the pyrimidine derivative of formula (I) is present in proportions of between 0.05 and 6% by weight relative to the total weight of the composition, preferably between 0.1 and 5%, and especially between 0.5 and 2% by weight.

**8.** Combination according to any one of Claims 1 to 7, characterised in that the physiologically acceptable medium for the components (A) and (B) is a medium consisting of water or a mixture of water and one or more organic solvents, or of a mixture of cosmetically or pharmaceutically acceptable organic solvents.

**9.** Combination according to any one of Claims 1 to 8, characterised in that at least one of the components (A) or (B) contains a solvent chosen from $C_1$-$C_4$ lower alcohols, alkylene glycols, and mono- and dialkylene glycol alkyl ethers.

**10.** Combination according to any one of Claims 1 to 9, characterised in that at least one of the components (A) or (B) is thickened by means of thickening or gelling agents.

**11.** Combination according to any one of Claims 1 to 10, characterised in that at least one of the components (A) or (B) also contains a cosmetically or pharmaceutically acceptable adjuvant chosen from preservatives, complexing agents, colorants, alkalinising or acidifying agents, anionic, cationic, nonionic or amphoteric surfactants as well as mixtures thereof, and anionic, cationic, nonionic or amphoteric polymers as well as mixtures thereof.

**12.** Process for cosmetic treatment of the hair or the scalp, characterised in that the components (A) and (B) are applied according to a combination as defined in any one of Claims 1 to 11, by means of a single composition or separately, either simultaneously or successively or separated by an interval of time, for the purpose of improving the appearance of the hair or the scalp.

**13.** Multi-compartment device or kit, characterised in that it comprises, in a first compartment, a component (A) as defined in any one of Claims 1 to 10, and a second compartment containing the component (B) as defined in any one of Claims 1 to 10.

**14.** Combination according to any one of Claims 1 to 10 for application as a medicinal product for the therapeutic treatment of hair loss.

**15.** Use of the combination according to any one of Claims 1 to 10 for the preparation of a medicinal product intended for use in the therapeutic treatment of hair loss.

## Patentansprüche

**1.** Mischung zum Induzieren und Stimulieren des Haarwuchses und zum Vermindern des Haarausfalles, dadurch **gekennzeichnet,** daß
sie umfaßt:
a) einen Bestandteil (A), enthaltend in einem physiologisch verträglichen Milieu mindestens einen Calcium-Antagonist und
b) einen Bestandteil (B), enthaltend in einem physiologisch verträglichen Milieu mindestens ein Pyrimidinderivat der Formel:

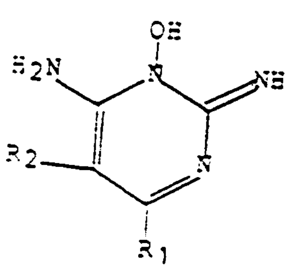

(I)

worin $R_1$ eine -

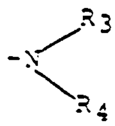

Gruppe darstellt,
wobei $R_3$ und $R_4$, unabhängig voneinander, Wasserstoff, eine Alkyl-, Alkenyl-, Alkylaryl-, Cycloalkylgruppe bedeuten, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-, Hexahydroazepinyl-, Heptamethylenimin-, Octamethylenimin-, Morpholin-und Alkyl-4-piperazidinylgruppen, wobei die heterozyklischen Gruppen an den Kohlenstoffatomen durch eine bis drei Niedrigalkyl-, Hydroxy- oder Alkoxygruppen substituiert sein können, und worin die $R_2$-Gruppe unter Wasserstoff, einer Alkyl-, Alkenyl-, Alkylalkoxy-, Cycloalkyl-, Aryl-, Alkylaryl-, Arylalkyl-, Alkylarylalkyl-, Alkoxyarylalkyl- oder Haloarylalkylgruppe ausgewählt ist, sowie die physiologisch verträglichen Säureadditionssalze davon, wobei die Bestandteile (A) und (B) Teile ein und derselben Zusammensetzung darstellen oder dazu bestimmt sind, auf behaarter Haut oder Haaren getrennt verwendet zu werden, entweder gleichzeitig oder aufeinanderfolgend oder nach einem Zeitablauf.

**2.** Mischung gemäß Anspruch 1,
dadurch **gekennzeichnet,** daß
die Antagonisten von Calcium aus Papaverinderivaten, 1,4-Dihydropyridinderivaten, Benzothiazepinderivaten, Cinnamylpiperazinderivaten, Nicergolin und Bepridil ausgewählt sind.

**3.** Mischung gemäß Anspruch 2,
dadurch **gekennzeichnet,** daß
die Papaverinderivate aus Verapamil, Gallopamil ausgewählt sind, daß die 1,4-Dihydropyridinderivate aus Nifedipin, Nicardipin, Nimodipin, Niludipin, Felodipin, Nitrendipin, Nisoldipin, Nilvadipin, Isradipin ausgewählt sind, daß das Benzothiazepinderivat aus Diltiazem ausgewählt ist und die Cinnamylpiperazinderivate aus Cinnarizin und Flunarizin ausgewählt sind.

**4.** Mischung gemäß eines jeden der Ansprüche 1 bis 3,
dadurch **gekennzeichnet,** daß
das Pyrimidinderivat der Formel (I) aus Verbindungen der Formel (I) ausgewählt ist, worin $R_2$ Wasserstoff bedeutet und $R_1$ eine Gruppe darstellt:

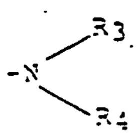

worin $R_3$ und $R_4$ einen Piperidinylring bilden, sowie der Salze davon.

**5.** Mischung gemäß Anspruch 4,
dadurch **gekennzeichnet,** daß
das Pyrimidinderivat der Formel (I) aus Amino-6-dihydro-1,2-hydroxy-1-imino-2-piperidino-4-pyrimidin oder "Minoxidil" zusammengesetzt ist.

**6.** Mischung gemäß eines jeden der Ansprüche 1 bis 5,
dadurch **gekennzeichnet,** daß
der Antagonist von Calcium im Bestandteil (A) in Mengen von 0,01 bis 10, vorzugsweise 0,01 bis 5, Gewichtsprozent und das Pyrimidinderivat der Formel (I) im Bestandteil (B) in Mengen von 0,05 bis 10, vorzugsweise 0,05 bis 5, insbesondere von 0,5 bis 4, Gewichtsprozent verwendet werden.

**7.** Mischung gemäß eines jeden der Ansprüche 1 bis 6,
dadurch **gekennzeichnet,** daß

die Bestandteile (A) und (B) in einer einzigen Zusammensetzung vorliegen, worin der Calcium-Antagonist in Mengen von 0,01 bis 5, vorzugsweise 0,05 bis 3, Gewichtsprozent, bezogen auf das Gesamtgewicht der Zusammensetzung, und das Pyrimidinderivat der Formel (I) in Mengen von 0,05 bis 6, vorzugsweise 0,1 bis 5, insbesondere 0,5 bis 2, Gewichtsprozent, bezogen auf Gesamtgewicht der Zusammensetzung, vorliegen.

**8.** Mischung gemäß eines jeden der Ansprüche 1 bis 7,
dadurch **gekennzeichnet,** daß
das physiologisch verträgliche Milieu für die Bestandteile (A) und (B) ein Milieu ist, das aus Wasser oder einer Mischung aus Wasser und eines oder mehrerer organischer Lösungsmittel oder aus einer Mischung aus kosmetisch oder pharmazeutisch verträglichen organischen Lösungsmitteln zusammengesetzt ist.

**9.** Mischung gemäß eines jeden der Ansprüche 1 bis 8,
dadurch **gekennzeichnet,** daß
mindestens einer der Bestandteile (A) oder (B) ein Lösungsmittel enthält, ausgewählt aus $C_1$-$C_4$-Niedrigalkoholen, Alkylenglykolen, Alkylethern von Mono- und Dialkylenglykol.

**10.** Mischung gemäß eines jeden der Ansprüche 1 bis 9,
dadurch **gekennzeichnet,** daß
mindestens einer der Bestandteile (A) oder (B) durch Verdickungs- oder Geliermittel verdickt ist.

**11.** Mischung gemäß eines jeden der Ansprüche 1 bis 10,
dadurch **gekennzeichnet,** daß
mindestens einer der Bestandteile (A) oder (B) auch einen kosmetisch oder pharmazeutisch verträglichen Hilfsstoff enthält, ausgewählt aus Konservierungs-, Komplexierungs-, Färbe-, alkalisch oder sauer machenden Mitteln, anionischen, kationischen, nicht-ionischen oder amphoteren oberflächenaktiven Mitteln, sowie deren Mischungen, anionischen, kationischen, nicht-ionischen oder amphoteren Polymeren, sowie deren Mischungen.

**12.** Verfahren zur kosmetischen Behandlung von Haaren oder von behaarter Haut,
dadurch **gekennzeichnet,** daß
man die Bestandteile (A) und (B) einer Mischung gemäß jeden der Ansprüche 1 bis 11 mittels einer einzigen Zusammensetzung oder in getrennter Weise, entweder gleichzeitig oder aufeinanderfolgend oder nach einem Zeitablauf, zur Verschönerung der Haare oder der behaarten Haut aufbringt.

**13.** Vorrichtung aus mehreren Teilen oder "Kit",
dadurch **gekennzeichnet,** daß
sie in einem ersten Teil einen Bestandteil (A), wie in jedem der Ansprüche 1 bis 10 definiert, und in einem zweiten Teil, enthaltend den Bestandteil (B), wie in jedem der Ansprüche 1 bis 10 definiert, umfassen.

**14.** Mischung gemäß eines jeden der Ansprüche 1 bis 10 zur Verwendung als Medikament zur therapeutischen Behandlung des Haarausfalles.

**15.** Verwendung der Mischung gemäß eines jeden der Anbsprüche 1 bis 10 zur Herstellung eines Medikaments zur Verwendung bei der therapeutischen Behandlung des Haarausfalles.